(19) Europäisches Patentamt
European Patent Office
Office européen des brevets

(11) **EP 4 339 273 A1**

(12) **EUROPEAN PATENT APPLICATION**
published in accordance with Art. 153(4) EPC

(43) Date of publication:
**20.03.2024 Bulletin 2024/12**

(21) Application number: **22807565.1**

(22) Date of filing: **13.05.2022**

(51) International Patent Classification (IPC):
**C12M 1/00** (2006.01)    **C12Q 1/04** (2006.01)

(52) Cooperative Patent Classification (CPC):
**C12M 1/00; C12Q 1/04**

(86) International application number:
**PCT/JP2022/020262**

(87) International publication number:
**WO 2022/239867 (17.11.2022 Gazette 2022/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.05.2021 JP 2021082676**

(71) Applicant: **Horiba Advanced Techno, Co., Ltd.**
**Kyoto-shi, Kyoto 6018551 (JP)**

(72) Inventors:
• **NAKAI, Yoko**
  **Kyoto-shi, Kyoto 601-8551 (JP)**
• **NAKAYAMA, Hideki**
  **Kyoto-shi, Kyoto 601-8551 (JP)**
• **FUKAO, Yoshiki**
  **Kyoto-shi, Kyoto 601-8551 (JP)**

(74) Representative: **Isarpatent**
**Patent- und Rechtsanwälte**
**Barth Hassa Peckmann & Partner mbB**
**Friedrichstraße 31**
**80801 München (DE)**

(54) **MICROORGANISM DETECTION APPARATUS AND MICROORGANISM DETECTION METHOD**

(57)    A microorganism detection apparatus detects contaminating microorganisms in a sample. For detecting the contaminating microorganisms more quickly than a conventional apparatus, the microorganism detection apparatus includes a pre-treatment unit that sorts out contaminating microorganisms from a sample and concentrates the contaminating microorganisms, and a measurement unit that measures an ATP content in a concentrate obtained as a result of concentration by the pre-treatment unit. The pre-treatment unit sorts out the contaminating microorganisms from the sample by removing cells other than the contaminating microorganisms in the sample by physical disruption, chemical disruption, and/or filtering.

FIG.1

EP 4 339 273 A1

**Description**

Technical Field

The present invention relates to an apparatus and a method for detecting contaminating microorganisms contained in a sample.

Background Art

For example, in a product containing cultured cells or the like, part thereof is collected as a sample at the time of shipment, and whether or not microorganism contamination has occurred is inspected.

As a method for detecting whether or not a sample contains contaminating microorganisms as a source of contamination in such an inspection, a method has been conceived in which a luminescent reagent is added to adenosine triphosphate (ATP) contained in microorganisms to measure bioluminescence thereof as exemplified by Patent Literature 1 and Patent Literature 2.

Direct measurement of ATP derived from contaminating microorganisms slightly contained in a sample is difficult. For this reason, in Patent Literature 1, the microorganisms are cultured, and the ATP content increased during the culture is measured. In Patent Literature 2, only microorganisms are separated from a sample using an antibody that specifically recognizes only microorganisms, and ATP taken out from the separated microorganism is amplified by an enzymatic reaction and then measured.

However, these methods require a step of amplifying microorganisms themselves or ATP derived from microorganisms, and thus have such a problem that it takes time to detect microorganisms.

Citation List

Patent Literatures

    Patent Literature 1: JP 2020-22374 A
    Patent Literature 2: JP 2006-81506 A

Summary of Invention

Technical Problem

The present invention has been made to solve the above problems, and a main object thereof is to detect microorganisms in a sample more quickly than in a conventional apparatus or method.

Solution to Problem

That is, a microorganism detection apparatus according to the present invention includes a pre-treatment unit that sorts out a contaminating microorganism from a sample and concentrates the contaminating microorganism, and a measurement unit that measures a content of ATP contained in a concentrate obtained as a result of concentration by the pre-treatment unit, in which the pre-treatment unit sorts out the contaminating microorganism from the sample by removing a cell other than the contaminating microorganism in the sample by physical disruption, chemical disruption, and/or filtering.

This microorganism detection apparatus measures the ATP content in the concentrate obtained as a result of concentration by removing cells other than contaminating microorganisms in the sample by physical disruption, chemical disruption and/or filtering. Thus, a step of amplifying contaminating microorganisms and ATP is unnecessary. As a result, contaminating microorganisms can be detected more quickly than in a conventional apparatus.

Examples of the physical disruption include those caused by adjusting the pressure or osmotic pressure of the sample, applying an ultrasonic wave or an electric shock to the sample, or bringing the sample into contact with beads for cell disruption or nanopillars.

Examples of the chemical disruption include those caused by adding, to the sample, one or more selected from a predetermined culture medium component, a surfactant, an enzyme, an apoptosis inducer, and a cytolysin, all of which can selectively kill a target cell, or by adjusting the temperature or pH of the sample to a temperature or pH that selectively kills a target cell.

Examples of the filtering include filtering using a filter that allows only the contaminating microorganisms to pass.

Examples of a specific aspect of the present invention include an aspect in which the measurement unit measures the content of ATP extracted from the contaminating microorganisms contained in the concentrate.

When the measurement unit is configured to measure the content of ATP extracted from the contaminating microorganisms contained in the concentrate after addition of an ATP scavenger for removing free ATP, only ATP derived from live contaminating microorganisms can be measured. In addition, the content of free ATP in the concentrate and the content of ATP derived from dead contaminating microorganisms can also be known by comparing this measurement result with the measurement result in the case where the ATP scavenger is not added.

When the contaminating microorganisms are in the form of highly durable spores, ATP may not be extracted by the ATP extraction solution. Given this factor, the measurement unit may be configured to measure the content of ATP extracted from the contaminating microorganisms contained in the concentrate after the addition of the germination liquid for germinating the contaminating microorganisms in the form of spores. With this configuration, ATP can be extracted from all the contaminating microorganisms including the contaminating microorganisms in the form of spores. By comparing with the measurement result in the case where the germination liquid is not added, the amount of contaminating microorganisms in the form of spores present in the concentrate can be known.

When the sample contains cultured cells, the effect of the present invention is particularly remarkably exhibited.

The contaminating microorganisms may form a biofilm, thus a biofilm decomposition unit that decomposes the biofilm is preferably further included.

A microorganism detection method according to the present invention includes sorting out the contaminating microorganism from the sample and concentrating the contaminating microorganism by removing a cell other than the contaminating microorganism in the sample by physical disruption, chemical disruption, and/or filtering, and measuring a content of ATP in a concentrate obtained by concentration.

When the method further includes a step of identifying the type of the contaminating microorganism contained in the concentrate, not only the presence of contaminating microorganisms but also the type of contaminating microorganisms can be identified.

Advantageous Effects of Invention

According to the present invention configured as described above, the ATP content in the concentrate obtained as a result of concentration by removing cells other than contaminating microorganisms in the sample by physical disruption, chemical disruption and/or filtering is measured. Thus, a step of amplifying contaminating microorganisms or ATP is unnecessary. As a result, contaminating microorganisms can be detected more quickly than in a conventional apparatus or method.

Brief Description of Drawings

FIG. 1 is a schematic view illustrating an overall configuration of a microorganism detection apparatus according to an embodiment of the present invention.

FIG. 2 is a schematic view illustrating a structure of a measurement unit included in the microorganism detection apparatus of the present embodiment.

FIG. 3 is a perspective view illustrating an appearance of the measurement unit included in the microorganism detection apparatus of the present embodiment.

FIG. 4 is a schematic view illustrating a measurement procedure in the measurement unit of the present embodiment.

FIG. 5 is a schematic view illustrating a structure of a pre-treatment unit of the present embodiment.

FIG. 6 is an enlarged sectional view of the pre-treatment unit of the present embodiment.

Description of Embodiment

Hereinafter, an embodiment of a microorganism detection apparatus according to the present invention will be described with reference to the drawings.

<Apparatus configuration>

A microorganism detection apparatus 100 of the present embodiment analyzes light generated by contaminating microorganism-derived substances contained in a sample to measure the amount of substances derived from such organisms. A description is given below for an ATP content measurement apparatus for measuring the content (amol (= $10^{-18}$ mol)) of adenosine triphosphate (ATP) as a contaminating microorganism-derived substance.

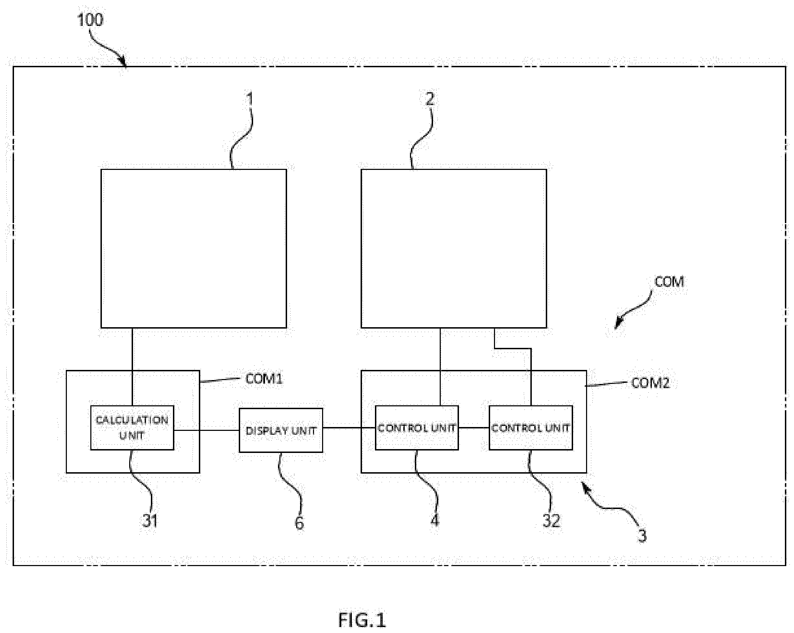

Specifically, as illustrated in FIG. 1, for example, the microorganism detection apparatus 100 includes: a pre-treatment unit 1 that pre-treats a sample; a measurement unit 2 that measures the ATP content in the treated liquid obtained as a result of treatment by the pre-treatment unit 1; a control unit 3 that controls the pre-treatment unit 1 and the measurement unit 2; a calculation unit 4 that calculates the ATP content based on an output signal from the measurement unit 2; and a display unit 6 that displays the ATP content calculated by the calculation unit 4. In the present embodiment, as an example of the control unit 3, a pre-treatment control unit 31 that controls the pre-treatment unit and a measurement control unit 32 that controls the measurement unit are included, but the present invention is not limited thereto.

The control unit 3, the calculation unit 4, and the like exhibit their functions with a computer COM that includes, for example, a CPU, a memory, an input/output interface, and an analog-to-digital (AD) converter. As the display unit 6, the display unit provided in the computer COM described above may be used, or a separately provided display or the like may be used. In FIG. 1, a COM1 functioning as the pre-treatment control unit 31 and a COM2 functioning as the measurement control unit 32, the calculation unit 4, and the like are provided, and only one display unit 6 is provided, but the present invention is not limited thereto.

Since the pre-treatment unit 1 has a characteristic configuration of the present invention, the pre-treatment unit 1 will be described in detail later.

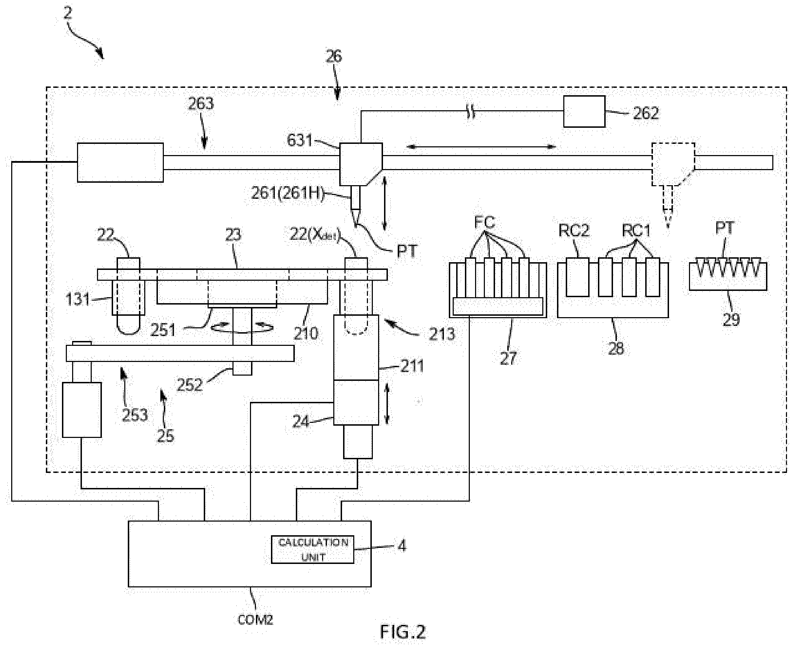

The measurement unit 2 is a so-called ATP meter. As illustrated in FIG. 2, for example, the measurement unit 2 includes: a holder 23 that holds a plurality of containers 22 for containing a sample; a photodetector 24 fixed at a predetermined position; a holder driving mechanism 25 that moves the holder 23; and a dispensing mechanism 26 that performs an action including dispensing, into the container 22 held by the holder 23, a luminescent reagent reacting with ATP to generate light.

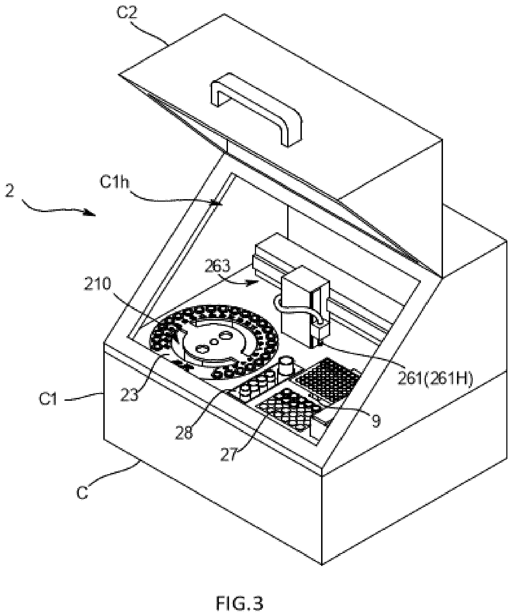

As illustrated in FIGS. 2 and 3, the measurement unit 2 according to the present embodiment includes a casing C having a door C2 for taking in/out the holder 23. The casing C includes: a casing main body C1 that accommodates measurement system equipment necessary for ATP measurement, such as the holder 23, the holder driving mechanism 25, and the dispensing mechanism 26; and the door C2 that is openable and closable and provided in the casing main body C1. A user can access the inside of the casing main body C1 by, for example, lifting the door C2 upward to be opened. The inside of the apparatus is brought into a dark room state by closing the door C2.

In addition, the casing main body C1 includes: a temperature control mechanism 27 that holds and controls the temperature of a plurality of specimen tubes FC containing a treated liquid obtained as a result of treatment by the pre-treatment unit 1; a reagent set part 28 in which reagent containers RC1, RC2 each containing a reagent are set; and a pipette tip set part 29 in which pipette tips PT used for the dispensing mechanism 26 are provided.

The reagent containers RC1 and the reagent container RC2 are set in the reagent set part 28. The reagent containers RC1 each contains a reaction reagent for extracting ATP from the treated liquid obtained as a result of pre-treatment by the pre-treatment unit 1. The reagent container RC2 contains the luminescent reagent. Examples of the reaction reagents include: an ATP scavenger that scavenges ATP (free ATP) other than live cells (live bacteria) contained in the treated liquid; a spore reaction solution for germinating bacteria in the form of spores; and an ATP extraction solution that extracts ATP from the live cells.

The holder 23 holds, for example, the plurality of containers 22 arranged circumferentially. Specifically, the holder 23 holds the plurality of containers 22 arranged on the same circle with respect to a predetermined rotation center. The container 22 includes a resin having a bottomed cylindrical shape. In the present embodiment, the container 22 includes a resin having a bottomed circular tube shape.

As illustrated in FIG. 2, the photodetector 24 detects light emitted from the sample in the container 22 held by the holder 23, and is, for example, a photomultiplier tube (PMT). The photodetector 24 is provided below the containers 22 held by the holder 23. An optical system including a reflector 211 for guiding light emitted from the sample in the container 22 to the photodetector 24 is provided above the photodetector 24.

The holder driving mechanism 25 moves the holder 23 to sequentially position the containers 22 held by the holder 23 at a detection position $X_{det}$ where detection is performed by the photodetector 24. Specifically, the holder driving mechanism 25 rotates the holder 23 around the predetermined rotation center. As illustrated in FIG. 2, the holder driving mechanism 25 includes: a mounting table 251 on which the holder 23 is mounted; a rotary shaft 252 for rotating the holder 23 mounted on the mounting table 251; and an actuator 253 for rotating the rotary shaft 252. In addition, the holder driving mechanism 25 is provided with a rotational position sensor (not illustrated) for detecting the rotational position of the holder 23. Based on the detection signal of the rotational position sensor, the actuator 253 is rotationally controlled so as to position the container 22 to be measured at the detection position $X_{det}$ by the control unit 3. A position serving as a mark such as a predetermined origin may be detected by the rotational position sensor, and other positions may be managed by using the number of pulses of the motor that is the actuator 253. Such a configuration is preferable because the configuration of the rotational position sensor can be simplified.

As illustrated in FIGS. 2 to 3, the dispensing mechanism 26 includes: a nozzle 261 for sucking or discharging the sample or each reagent; a pump mechanism 262 that drives the suction or discharge of the nozzle 261 via a flow path connected to the nozzle 261, such as a syringe; and a nozzle moving mechanism 263 that moves the nozzle 261 in a predetermined direction.

The nozzle 261 includes a tip holder 261H for attachably and detachably holding the pipette tip PT for coming into contact with the sample or each reagent and holding it. The tip holder 261H includes an internal flow path formed therein. The flow path is connected to the proximal end portion of the tip holder 261H, and the pipette tip PT is connected to the distal end opening of the tip holder 261H.

The nozzle moving mechanism 263 linearly moves the nozzle 261 in the horizontal direction (an X-axis direction and a Y-axis direction), as well as linearly moves the nozzle 261 in the vertical direction (a Z-axis direction). The nozzle moving mechanism 263 is controlled by the control unit 3 described above.

The pipette tip PT having been used for the dispensation is detached at the position above a disposal box 210 of the holder 23. The disposal box 210 is, for example, provided integrally with the holder 23. In the present embodiment, the disposal box 210 is provided on the inside of the plurality of containers 22, which is a dead space in the holder 23. Specifically, the detachment of the pipette tip PT may be performed by moving the nozzle 261 to a tip detachment member (not illustrated) disposed above the disposal box 210. Alternatively, the detachment may be performed by providing a tip detachment member at a movable member 631, moving the movable member 631 to a position above the disposal box 210, and then using the tip detachment member.

As illustrated in FIG. 2, the measurement unit 2 may further include a light shielding mechanism 213. The light shielding mechanism 213 guides light emitted from the sample in the container 22 at the detection position $X_{det}$ to the photodetector 24 and prevents light emitted from the samples in the other containers 22 (specifically, the container 22 in which the measurement is completed) from being guided to the photodetector 24.

<Analysis method>

Next, a method for detecting microorganisms will be described together with the operation of the microorganism detection apparatus 100 configured as described above.

The specimen tube FC containing the treated liquid obtained as a result of pre-treatment by the pre-treatment unit is set to the temperature control mechanism 27. The door C2 is closed while a predetermined number of the specimen tubes FC are set, and the measurement is started. Although each container 22 held by the holder 23 in this state is empty, the container 22 for measuring a standard solution contains a standard solution having a known ATP content.

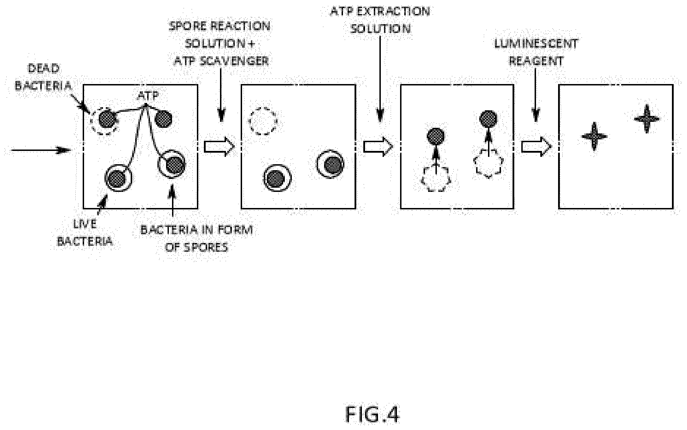

When the measurement is started, the control unit 3 causes the dispensing mechanism 26 to dispense each reaction reagent into the specimen tubes FC held by the temperature control mechanism 27, for example, in accordance with a predetermined sequence as illustrated in FIG. 4. In this manner, predetermined processing (ATP extraction) is performed on the sample in the specimen tube FC. The pipette tip PT is replaced for each reaction reagent, and the used pipette tip PT is disposed of in the disposal box 210.

Specifically, a mixed liquid of the ATP scavenger and the spore reaction solution is dispensed into the samples in the specimen tubes FC, and the specimen tubes FC are kept at a predetermined temperature and waiting is performed until the reaction of each reagent is completed. Thereafter, the ATP extraction solution is dispensed into the samples in the specimen tubes FC, and the specimen tubes FC are kept at a predetermined temperature and waiting is performed until the extraction of ATP is completed. The ATP scavenger and the spore reaction solution may be separately dispensed instead of dispensing the mixed liquid of the ATP scavenger and the spore reaction solution.

For example, as illustrated in FIG. 4, an ATP scavenger and a spore reaction solution are added to the treated liquid, then ATP is extracted with the ATP extraction solution, then a luminescent reagent is added thereto, and measurement is performed. This procedure enables measurement of the content of ATP derived from the entire contaminating microorganisms in a live state including the contaminating microorganisms in the form of spores. The content of ATP extracted from a control sample to which the spore reaction solution is not added at this time may also be measured. When do so, the content of ATP derived only from the microorganism in the form of spores can be calculated.

For the calibration liquid, during the waiting time after the reagent is dispensed into the sample as described above, each reaction reagent is dispensed in accordance with a predetermined sequence into a standard solution whose ATP content is known and a zero solution whose ATP content is zero. Specifically, after the ATP scavenger and the spore reaction solution are dispensed into each of a container for standard solution measurement and a container for blank measurement, the ATP extraction solution is dispensed thereinto. Thereafter, the standard solution or the zero solution is dispensed thereinto. The order of dispensing each reaction reagent into the zero solution is not limited to the above.

At this time, for example, the following are set to be identical to one another: a mixing ratio of the sample, the ATP scavenger, the spore reaction solution, and the ATP extraction solution; a mixing ratio of the standard solution, the ATP scavenger, the spore reaction solution, and the ATP extraction solution; and a mixing ratio of the zero solution, the ATP scavenger, the spore reaction solution, and the ATP extraction solution. Specifically, these mixing ratios are set to a predetermined value. By making the liquid amount of each reagent identical among the sample, the standard solution, and the zero solution in this manner, the pH in the liquids can be made identical. As a result, the prerequisites of the liquids before the luminescent reagent is dispensed can be made the same, and light intensity can be accurately detected.

Thereafter, the dispensing mechanism 26 aliquots the pre-treated sample in each specimen tube FC and dispenses the sample into the respective one of containers 22 held by the holder 23.

Then, the control unit 3 causes the holder driving mechanism 25 to move the container 22 to be measured to the detection position $X_{det}$. After the container 22 to be measured is moved to the detection position $X_{det}$, the control unit 3 causes the dispensing mechanism 26 to introduce the luminescent reagent into the container 22 at the detection position $X_{det}$. In this manner, light emitted from the sample in the container 22 at the detection position $X_{det}$ is detected by the photodetector 24, whereby luminescence is measured. Before the luminescence measurement of each container 22, blank measurement and standard solution measurement are performed, and zero point calibration and span calibration are performed.

The light intensity signal obtained by the photodetector 24 is subjected to arithmetic processing by the calculation unit 4 to calculate an ATP content (amol).

Specifically, the calculation unit 4 subtracts the "light intensity signal obtained before the addition of the luminescent reagent" from the "light intensity signal obtained after the addition of the luminescent reagent to the sample" to remove the accumulation of light in the container 22. Through this process, a value related to the amount of the organism-derived substances is calculated.

The "light intensity signal obtained after the addition of the luminescent reagent to the sample" is an integrated average signal that is an average value of integrated signals for a predetermined time (for example, several seconds to several tens of seconds) from the time point when the luminescent reagent is introduced. The "light intensity signal obtained before the addition of the luminescent reagent" is an integrated average signal that is an average value of integrated signals for a predetermined time (for example, several seconds to several tens seconds) before the luminescent reagent is introduced. Here, the "light intensity signal obtained before the addition of the luminescent reagent" is based on the light accumulated in the container 22. For example, if the container 22 is placed outside the microorganism detection apparatus 100 before the start of measurement, light such as ultraviolet light or fluorescent light may be accumulated in the container 22. Given this factor, the calculation unit 4 subtracts a "second light intensity signal that is the light intensity signal derived only from the container 22" from a "first light intensity signal including the light intensity signal derived from the container 22 and the organism-derived light intensity signal". With this operation, the microorganism detection apparatus 100 can accurately calculate only the light intensity signal derived from the contaminating microorganisms. The same applies to signal processing in blank measurement and standard solution measurement. The light intensity signal is not limited to the integrated average signal. The light intensity signal may be a simple integrated signal for a predetermined time (for example, several seconds to several tens of seconds) from the time point when the luminescent reagent is introduced or may be a signal subjected to other arithmetic processing.

In this manner, the calculation unit 4 calculates the ATP content (amol) for each container 22 by the following equation (Mathematical Expression 1).

[Mathematical Expression 1]

$$ATP[amol] = \frac{Sample_{signal} - Zero_{signal}}{STD_{signal} - Zero_{signal}} \times 1000$$

$Sample_{signal}$ is a signal obtained by sample measurement and is a signal obtained by subtracting the "light intensity signal obtained before the addition of the luminescent reagent to the sample" from the "light intensity signal obtained after the addition of the luminescent reagent to the sample".

$STD_{signal}$ is a signal obtained by standard solution measurement and is a signal obtained by subtracting the "light intensity signal obtained before the addition of the luminescent reagent to the standard solution" from the "light intensity signal obtained after the addition of the luminescent reagent to the standard solution".

$Zero_{signal}$ is a signal obtained by blank measurement and is a signal obtained by subtracting the "light intensity signal obtained before the addition of the luminescent reagent to the zero solution" from the "light intensity signal obtained after the addition of the luminescent reagent to the zero solution". In the blank measurement, a luminescence peak is generated when a luminescent reagent is added, but the luminescence peak may not be generated.

The above-described calculation can remove variations in the light accumulation amount of each of the container 22 for standard solution measurement, the container 22 for blank measurement, and the container 22 for sample measurement to accurately calculate the ATP content.

A user determines whether or not contaminating microorganisms are present in the sample based on the ATP content calculated as described above. Specifically, for example, a predetermined threshold value may be set for the ATP concentration in the sample obtained from the concentration factor of the sample in the pre-treatment unit and the calculated ATP content. When the ATP concentration in the sample exceeds this threshold value, it may be determined that contaminating microorganisms are present.

After the luminescence measurement of one container 22 is completed, the control unit 3 causes the holder driving mechanism 25 to move the next container 22 to be measured to the detection position $X_{det}$. In this way, the luminescence measurement of the sample in each container 22 is sequentially performed. The pipette tip PT is replaced for each time of luminescence measurement of the sample in the container 22, and the used pipette tip PT is disposed of in the disposal box 210. When the risk of contamination is low, such as when the same reagent is continuously dispensed, the pipette tip PT may be used without being replaced each time.

After the measurement is completed for all the samples in this manner, the door C2 is opened to replace the specimen tubes FC held by the temperature control mechanism 27 as well as to replace the containers 22 held by the holder 23. Here, when the containers 22 held by the holder 23 are replaced, the holder 23 is dismounted from the apparatus main body by holding the holding hole 3h of the holder 23. In the holder 23, the pipette tips PT having been used and disposed of are in the disposal box 210 of the holder 23. Thus, by dismounting the holder 23 from the apparatus main body, the pipette tip PT having been disposed of can also be taken out from the apparatus main body at the same time.

<Characteristic configuration>

The pre-treatment unit 1 has, for example, the following functions: a function as a sorter that sorts out contaminating microorganisms as a detection target from a sample obtained by sampling a part of a product such as cultured cells; and a function as a concentrator that obtains a concentrate obtained by concentrating the sorted contaminating microorganisms.

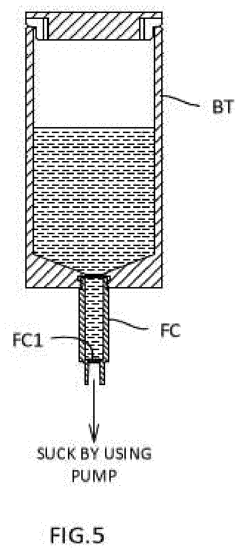

For example, the pre-treatment unit 1 removes cultured cells or the like by disruption and/or filtering to sort out only contaminating microorganisms. The disruption may be performed in a bottle BT that stores a large volume of sample as illustrated in FIG. 5. The filtering may be performed using a filter (not illustrated) or the like impermeable to the cultured cells and permeable to only contaminating microorganism cells.

In addition, the pre-treatment unit 1 uses a filter FC1 impermeable to the contaminating microorganisms and concentrates the contaminating microorganisms sorted out as described above. For example, as illustrated in FIG. 5, the pre-treatment unit 1 includes: the bottle BT that stores a large volume of sample; a cartridge (specimen tube FC) that is attachably and detachably mounted to a lower end opening of the bottle BT and includes the filter FC1 therein in which a fine hole sized not to allow contaminating microorganisms to permeate is formed; and a pump (not illustrated) that sucks liquid inside the specimen tube from a lower end portion of the specimen tube. The sample can be concentrated on the filter FC1 of the specimen tube FC by sucking the liquid inside the specimen tube FC from the lower end portion of the specimen tube FC by using a pump or the like while the upper end portion of the cartridge is inserted into the lower end opening of the bottle BT. The operation of the pump or the like may be controlled by the pre-treatment control unit 31 described above.

Since the filter FC1 is permeable to the cell debris generated when the cells other than the contaminating microorganisms are disrupted, only the cells of the contaminating microorganisms are substantially concentrated on the filter FC1.

In order to remove cell debris other than contaminating microorganism cells as much as possible, the pre-treatment unit 1 may wash the concentrate by adding distilled water, an appropriate buffer, or the like onto the filter FC1. This washing step may be performed by the pre-treatment unit 1 or may be manually performed by a user himself/herself.

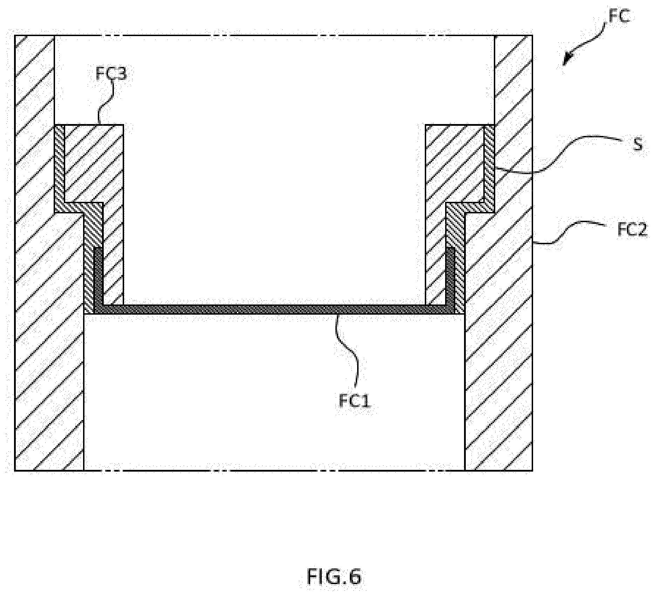

For example, as illustrated in FIG. 6, the above-described specimen tube FC includes: a tubular tube main body FC2 having both ends opened; and a filter cassette FC3 disposed so as to be in contact with an inner peripheral surface of the tube main body FC2 and disposing the above-described filter FC1 in the middle of the tube main body FC2 in a direction perpendicular to the axial direction of the tube main body FC2. When the filter cassette FC3 is mounted to the inner peripheral surface of the tube main body FC2, a slight space S may be formed between the filter cassette FC3 and the inner peripheral surface of the tube main body FC2. Upon the supply of a sample into the specimen tube FC while the space S being formed, if the sample contains a component that may affect luminescence measurement, accurate measurement may be hindered once a part of the sample enters the space S.

In view of this, a buffer, distilled water, or the like that does not affect ATP measurement is preferably allowed to flow and enter in advance into the space S formed between the above-described filter cassette FC3 and tube main body FC2 before the sample is supplied to the specimen tube FC. In order to make it easier for these liquids to enter the space described above, a chemical agent such as a surfactant may be added to a buffer or distilled water.

<Pre-treatment method>

Examples of the means by which the pre-treatment unit 1 configured as described above sorts out contaminating microorganisms as a detection target from a sample include a method of disrupting cells other than contaminating microorganisms in a sample by a physical or chemical method and a method of filtering such cells.

Examples of the physical disruption include those caused by adjusting the pressure or osmotic pressure of the sample, applying an ultrasonic wave or an electric shock to the sample, or bringing the sample into contact with beads for cell disruption or nanopillars, both of which selectively disrupt cells depending on the size.

Example of "adjusting the pressure or osmotic pressure of the sample" include a method of disrupting cells other than contaminating microorganisms as a detection target by adjusting the water pressure or osmotic pressure under which contaminating microorganisms can survive but other cells cannot survive. In particular, cultured cells or the like obtained by culturing cells of a multicellular organism have lower resistance to pressure change and osmotic pressure than cells of a unicellular organism such as a microorganism. Thus, this method can easily sort out only contaminating microorganisms.

Similarly, in a case where an ultrasonic wave or an electric shock is applied to a sample, contaminating microorganisms as a detection target are sorted out by applying an ultrasonic wave or an electric shock in a range in which contaminating microorganisms can survive but other cells are disrupted. In particular, since cultured cells and the like have a larger cell size than cells of microorganisms, cultured cells and the like are more susceptible to an ultrasonic wave or an electric shock than microorganisms. According to experiments conducted by the inventors of the present invention, it has been confirmed that, for example, in the case of electric shock, cultured cells are disrupted at a voltage of 1 kV/cm or less, but microorganisms are not disrupted.

Beads for cell disruption suitable to the size and type of cells of a disruption target are commercially available, and the size and material of beads used for cell disruption are completely different between beads for microorganisms and beads for other cells. For this reason, by selecting beads having an optimum size or material for disrupting cells other than contaminating microorganisms, for example, cultured cells of multicellular animals, cells other than the contaminating microorganisms can be disrupted without disrupting the contaminating microorganisms. When cells are disrupted by beads, the shaking speed and shaking time differ depending on the target cells. Thus, only cells other than contaminating microorganisms can also be disrupted not only by adjusting the size and material of the beads but also by adjusting the shaking speed and shaking time.

Alternatively, by bringing the sample into contact with the nanopillars having a fine needle-like structure, only cells having a relatively large size may be disrupted with the needle-like structure of the nanopillars.

Examples of the chemical disruption include those caused by adding, to the sample, one or more selected from a predetermined culture medium component, a surfactant, an enzyme, an apoptosis inducer, and a cytolysin, or by adjusting the temperature or pH of the sample.

As for the chemical disruption, any method can be adopted as long as contaminating cells as a detection target can survive but other cells cannot survive, as in the physical disruption described above. For example, the composition of the sample may be adjusted by adding a predetermined culture medium component to the sample. As another example, the concentration of the surfactant in the sample may be adjusted by controlling the amount of the surfactant to be added to the sample. Alternatively, a protease that can specifically disrupt other cells without disrupting the cells of the contaminating microorganisms as a detection target, or a toxin such as cytolysin may be added to the sample.

As for the surfactant, study was conducted for the conditions for cell disruption by various types of surfactants after adjustment of the concentration of the surfactant in the sample to be equal to or higher than the critical micelle concentration. As a result, it was confirmed that the concentration of the surfactant required for disruption varies depending on the size of the cells, for example, larger cells are disrupted by a lower concentration of the surfactant. Among them, as a surfactant, use of a nonionic surfactant that is a nonpolar surfactant is considered to be preferable. This is because the action on cells is mild as compared with an anionic surfactant, a cationic surfactant, and an amphoteric surfactant that have polarity, and conditions for selectively disrupting cells can be easily set.

For other approaches, the temperature and pH of the sample may be set in such a range that contaminating microorganisms can survive but other cells die, or indirect disruption may be performed through autolysis by making other cells into dead cells with an apoptosis inducer or the like.

For example, when the cells other than contaminating microorganisms present in a sample are cells of a multicellular animal, a temperature range and pH range in which the cells of the multicellular animal can survive are generally narrower than those of the microorganisms. Therefore, cells other than contaminating microorganisms can be made dead cells by setting the conditions (temperature and/or pH) to a range in which only the contaminating microorganisms can survive. It is necessary to prevent microorganism contamination derived from the experimental instrument or the like by reducing the number of experimental instruments to be in contact with the sample solution as much as possible. To such ends, for example, when the pH of the sample solution is changed to a target pH range, the pH is preferably not adjusted by a titrimetry. Instead, measures are preferably taken such that the requisite amount of reagents is calculated in advance and then these reagents are added at a time.

When the main component of the sample is a buffer, it is preferable to use a buffer that easily changes the pH within a desired pH range, particularly within a range of pH 5.5 or more and pH 8.5 or less. Examples of such buffers include 2-amino-2-hydroxymethyl-1,3-propanediol (Tris), phosphate-buffered saline (PBS), 2-morpholinoethanesulfonic acid, monohydrate (MES), bis(2-hydroxyethyl)iminotris(hydroxymethyl)methane (Bis-Tris), N-(2-acetamido)iminodiacetic acid (ADA), piperazine-1,4-bis(2-ethanesulfonic acid)(PIPES), N-(2-acetamido)-2-aminoethanesulfonic acid (ACES), 3-morpholinopropanesulfonic acid (MOPSC), N,N-bis(2-hydroxyethyl)-2-aminoethanesulfonic acid (BES), 3-morpholinopropanesulfonic acid (MOPS), N-tris(hydroxymethyl)methyl-2-aminoethanesulfonic acid (TES), 2-[4-(2-hydroxyethyl)-1-piperazinyl]ethanesulfonic acid (HEPES), 3-[N,N-bis(2-hydroxyethyl)amino]-2-hydroxypropanesulfonic acid (DIPSO), 2-hydroxy-N-tris(hydroxymethyl)methyl-3-aminopropanesulfonic acid (TAPSO), and piperazine-1,4-bis(2-hydroxy-3-propanesulfonic acid),dehydrate (POPSO).

As for the apoptosis inducer, an apoptosis inducer that acts only on cells other than contaminating microorganisms as a detection target and causes apoptosis without acting on the contaminating microorganisms can be selected from known apoptosis inducers and used.

Examples of the known apoptosis inducers that cause apoptosis include a wide variety of substances such as chemically synthesized substances, naturally occurring substances, and organism-derived substances, and research on action mechanisms thereof has progressed. Therefore, no difficulty may be encountered in selecting an apoptosis inducer that acts only on cells other than contaminating microorganisms without acting on contaminating microorganisms.

Only one of the physical disruption method and the chemical disruption method described above may be selected and used, or these methods may be used in combination.

As the filtering, for example, a filter that allows only contaminating microorganisms to pass is conceivably used. In this case, it is conceivable to use a size exclusion filter that does not allow cells other than contaminating microorganisms, for example, cultured cells larger than the contaminating microorganisms to pass and allows only contaminating microorganisms smaller in size than the cultured cells, unlike the above-described filter used for concentrating the contaminating microorganisms. This method is particularly effective when cells having completely different cell sizes, such as cultured cells and contaminating microorganisms, are separated from each other.

The step of sorting out contaminating microorganisms as described above may be performed inside a tank or the like prepared separately from the bottle BT described above. A part of the pre-treatment step may be manually performed by a user himself/herself or may be automatically performed by the pre-treatment unit 1.

<Effects of present embodiment>

According to the microorganism detection apparatus 100 of the present embodiment configured as described above, the ATP content in the concentrate obtained by sorting out the contaminating microorganisms as a detection target from the sample and then concentrating the contaminating microorganisms is measured. Thus, even when the number of contaminating microorganisms in the sample is very small, the content of ATP derived from the microorganisms can be measured by a conventional ATP meter.

Therefore, steps such as culturing microorganisms and amplifying ATP extracted from microorganisms are unnecessary, and contaminating microorganisms can be detected more quickly than in a conventional apparatus.

Furthermore, in the present embodiment, the measurement unit subtracts the light intensity signal obtained before the reaction of the sample and the luminescent reagent from the light intensity signal obtained after the reaction of the sample and the luminescent reagent to calculate the value related to the content of ATP. Thus, it is not necessary to detect the "luminescence intensity in a state where the luminescence ceases" as in a conventional apparatus. As a result, the measurement time of the sample can be shortened. Consequently, the time required for detecting contaminating microorganisms can further be shortened.

The method for sorting out contaminating microorganisms from a sample is based on: physical disruption caused by adjusting the temperature or osmotic pressure of the sample or applying an ultrasonic wave or an electric shock to the sample; chemical disruption caused by adding one or more selected from a surfactant, an enzyme, and a cytolysin to the sample; filtering; or the like. Therefore, only contaminating microorganisms can be easily concentrated without preparing a special reagent or a special apparatus.

< Other embodiments>

Note that the present invention is not limited to the above embodiment.

For example, in the above-described embodiment, the luminescent reagent is added to the container containing the biological sample, but the biological sample may be added to the container containing the luminescent reagent.

In addition, identification may be performed on the species of bacteria contained in the sample for which ATP has been measured by the measurement unit of the above-described embodiment.

Specifically, it is conceivable to identify the species of bacteria from deoxyribonucleic acid (DNA) or ribonucleic acid (RNA) contained in the residual liquid by using the residual liquid after adding the luminescent reagent (sample after ATP measurement) or the residual liquid in the specimen tube FC (sample before ATP measurement). More specifically, the species of bacteria are identified from the residual liquid using a DNA sequencer. In the case of RNA, a DNA sequencer can be used after DNA is synthesized by reverse transcription.

As processing for analysis with a DNA sequencer, DNA is conceivably amplified by a DNA amplification method (PCR). In this case, DNA is collected using, for example, DNA collecting beads from the residual liquid, and the collected DNA is amplified by PCR. The residual liquid contains an ATP extraction solution.

As the ATP extraction solution, for example, a surfactant, a mixed liquid of ethanol and ammonia, methanol, ethanol, trichloroacetic acid, perchloric acid, and a Tris buffer can be suitably used. Examples of the surfactant include sodium dodecyl sulfate, potassium lauryl sulfate, sodium monolauroyl phosphate, sodium alkylbenzenesulfonate, benzalkonium chloride, benzethonium chloride, cetylpyridinium chloride, cetyltrimethylammonium bromide, and myristyldimethylbenzylammonium chloride. Some ATP extraction solutions inhibit the function of a DNase. When PCR is performed on a sample after ATP measurement, the ATP extraction solution may inactivate the enzyme of PCR. Therefore, the ATP extraction solution may be removed, diluted, neutralized, or masked before PCR.

It is also considered that contaminating microorganisms present in the sample may form a biofilm. Efficient extraction of ATP from contaminating microorganisms protected by such a biofilm may be difficult. In view of this, the microorganism detection apparatus may further include a biofilm decomposition unit that decomposes the biofilm in a sample, for example, in a preceding stage of the pre-treatment unit 1.

Examples of a specific configuration of the biofilm decomposition unit include a filtration apparatus using a track-etched membrane, a nylon mesh, or the like.

In the bottle BT described above, a biofilm-decomposing enzyme or the like may be added to chemically decompose a biofilm. Alternatively, the biofilm may be decomposed by reciprocating the sample in the thin tube to apply shear stress or the like. The biofilm decomposition step may be performed by a decomposition unit included in the microorganism detection apparatus or may be manually performed by a user.

Furthermore, the ATP content of bacteria in the form of spores (spore bacteria) can also be measured by the microorganism detection apparatus of the embodiment. That is, the ATP content of the spore bacteria can be measured by subtracting the ATP content before the spore bacteria germinate from the ATP content after the spore bacteria germinate.

Specifically, the ATP content (Y [amol]) of only bacteria (live bacteria) in a normal state before germination of the spore bacteria can be measured by performing ATP measurement without adding the spore reaction solution to the treated liquid (concentrate) or before the spore bacteria germinate when the spore reaction solution is added. The ATP content (X [amol]) of both the spore bacteria and the live bacteria can be measured by performing ATP measurement after the spore reaction solution of the sample is added and the spore bacteria is germinated. Then, the ATP content of the spore bacteria can be calculated by X - Y [amol]. When the value of X - Y is large, it is found that spore bacteria are generated, and the user can take measures such as cleaning using a sporicidal agent. The ATP content of spore bacteria can also be measured by germinating spore bacteria through heating or addition of nutrients after killing live bacteria in a sample using a certain method (e.g., a heat shock method).

Furthermore, in the same manner, in a case where the ATP scavenger is added to the treated liquid and a case where the ATP scavenger is not added to the treated liquid, ATP is extracted by using the ATP extraction solution, and measurement may be performed by adding the luminescent reagent. In this manner, ATP derived from dead cells of microorganisms and free ATP not derived from microorganisms in the treated liquid can be eliminated, and thus the content of ATP derived from only live microorganisms can be calculated. Estimation of the abundance ratio between dead microorganisms and live microorganisms, or the like can also be performed by comparison with the case where ATP scavenger is not added.

Determination as to whether or not the microorganism is present in the sample may be performed by a user as described above. Alternatively, for such purposes, a determination unit that determines whether or not the microorganism is present from the ATP content calculated by the calculation unit may further be included.

In addition, the present invention is not limited to the embodiment described above, and it goes without saying that various modifications can be made without departing from the spirit of the present invention.

Industrial Applicability

According to the present invention, microorganisms in a sample can be detected more quickly than in a conventional apparatus or method.

Reference Signs List

100    microorganism detection apparatus
1        pre-treatment unit

2       measurement unit

**Claims**

1.  A microorganism detection apparatus that detects a contaminating microorganism in a sample, the microorganism detection apparatus comprising:

    a pre-treatment unit that sorts out a contaminating microorganism from a sample and concentrates the contaminating microorganism; and
    a measurement unit that measures a content of adenosine triphosphate (ATP) in a concentrate obtained as a result of concentration by the pre-treatment unit,
    wherein the pre-treatment unit sorts out the contaminating microorganism from the sample by removing a cell other than the contaminating microorganism in the sample by physical disruption, chemical disruption, and/or filtering.

2.  The microorganism detection apparatus according to claim 1, wherein the physical disruption is caused by adjusting a pressure or an osmotic pressure of the sample, applying an ultrasonic wave or an electric shock to the sample, or bringing the sample into contact with a bead for cell disruption or a nanopillar.

3.  The microorganism detection apparatus according to claim 1, wherein the chemical disruption is caused by adding, to the sample, one or more selected from a predetermined culture medium component, a surfactant, an enzyme, an apoptosis inducer, and a cytolysin, or by adjusting a temperature or pH of the sample to a temperature or pH that selectively kills a target cell.

4.  The microorganism detection apparatus according to claim 1, wherein the filtering uses a filter that allows only the contaminating microorganism to pass.

5.  The microorganism detection apparatus according to any one of claims 1 to 4, wherein the measurement unit measures a content of ATP extracted from the contaminating microorganism in the concentrate.

6.  The microorganism detection apparatus according to any one of claims 1 to 4, wherein the measurement unit measures a content of ATP extracted from the contaminating microorganism in the concentrate after the addition of an ATP scavenger that removes free ATP.

7.  The microorganism detection apparatus according to any one of claims 1 to 4, wherein the measurement unit measures a content of ATP extracted from the contaminating microorganism in the concentrate after the addition of a spore reaction solution that germinates the contaminating microorganism in a form of a spore.

8.  The microorganism detection apparatus according to any one of claims 1 to 7, wherein the sample contains a cultured cell as a cell other than the contaminating microorganism.

9.  The microorganism detection apparatus according to any one of claims 1 to 7, further comprising a biofilm decomposition unit that decomposes a biofilm, wherein
    the biofilm decomposition unit is provided in a preceding stage of the pre-treatment unit.

10. A microorganism detection method for detecting a contaminating microorganism in a sample, the microorganism detection method comprising:
    sorting out the contaminating microorganism from the sample and concentrating the contaminating microorganism by removing a cell other than the contaminating microorganism in the sample by physical disruption, chemical disruption, and/or filtering; and measuring an ATP content in a concentrate obtained by concentration.

11. The microorganism detection method according to claim 10, further comprising identifying a type of a contaminating microorganism contained in the concentrate.

FIG.1

FIG.2

FIG.3

FIG.4

BT

FC

FC1

SUCK BY USING
PUMP

FIG.5

FIG.6

## INTERNATIONAL SEARCH REPORT

| | International application No. |
|---|---|
| | **PCT/JP2022/020262** |

| | |
|---|---|
| **A.** | **CLASSIFICATION OF SUBJECT MATTER** |

*C12M 1/00*(2006.01)i; *C12Q 1/04*(2006.01)i
FI: C12M1/00 A; C12Q1/04

According to International Patent Classification (IPC) or to both national classification and IPC

| | |
|---|---|
| **B.** | **FIELDS SEARCHED** |

Minimum documentation searched (classification system followed by classification symbols)

C12M1/00; C12Q1/04

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII), CAplus/MEDLINE/EMBASE/BIOSIS (STN)

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| X | JP 2008-516632 A (MEDICAL INNOVATIONS INTERNATIONAL, INC.) 22 May 2008 (2008-05-22) | 1-5, 10, 11 |
| | abstract, claims, paragraphs [0009]-[0011], etc. | |
| Y | abstract, claims, paragraphs [0009]-[0011], etc. | 1-11 |
| X | JP 10-028599 A (TOYO INK MFG CO., LTD.) 03 February 1998 (1998-02-03) | 1-5, 10, 11 |
| | abstract, claims, paragraphs [0002]-[0004], etc. | |
| Y | abstract, claims, paragraphs [0002]-[0004], etc. | 1-11 |
| Y | 西川圭祐ほか. 製薬用水における微生物迅速試験法の適用と活用事例. PHARM TECH JAPAN. March 2021, vol. 37, no. 3, pp. 417-425, non-official translation (NISHIKAWA, Keisuke et al. Case Study of Application and Utilization of Microorganism Rapid Test Method in Pharmaceutical Water.) pp. 62, 63, "4-1. Device Overview and Measurement Principles" | 1-11 |
| Y | JP 2010-099052 A (OLYMPUS CORP.) 06 May 2010 (2010-05-06) abstract, paragraphs [0006]-[0008], etc. | 1-11 |

☐ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **22 June 2022** | **05 July 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
**Information on patent family members**

International application No.

**PCT/JP2022/020262**

| Patent document cited in search report | | | Publication date (day/month/year) | Patent family member(s) | | Publication date (day/month/year) |
|---|---|---|---|---|---|---|
| JP | 2008-516632 | A | 22 May 2008 | US 2006/0084127 A1 abstract, claims, paragraphs [0010]-[0012], etc. WO 2006/044895 A1 EP 1812584 A1 | | |
| JP | 10-028599 | A | 03 February 1998 | US 5908751 A abstract, claims, column 1, 1st paragraph to column 2, 3rd paragraph, etc. EP 803577 A2 | | |
| JP | 2010-099052 | A | 06 May 2010 | (Family: none) | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020022374 A
- JP 2006081506 A